(19) Europäisches Patentamt
European Patent Office
Office européen des brevets

(11) EP 4 800 115 A1

(12) EUROPEAN PATENT APPLICATION
published in accordance with Art. 153(4) EPC

(43) Date of publication:
**02.09.2026 Bulletin 2026/36**

(21) Application number: **24897560.9**

(22) Date of filing: **26.11.2024**

(51) International Patent Classification (IPC):
***C12N 15/90*** (2006.01) ***C12N 5/10*** (2006.01)
***C12P 21/00*** (2006.01) ***C12N 15/12*** (2006.01)
***C12N 15/85*** (2006.01)

(52) Cooperative Patent Classification (CPC):
**C12N 5/10; C12N 15/90; C12P 21/00;**
C07K 14/435; C12N 15/85

(86) International application number:
**PCT/JP2024/041881**

(87) International publication number:
**WO 2025/115876 (05.06.2025 Gazette 2025/23)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority: **29.11.2023 JP 2023202229**

(71) Applicant: **FUJIFILM Corporation**
**Tokyo 106-8620 (JP)**

(72) Inventors:
- **ONODERA, Keiichi**
**Ashigarakami-gun, Kanagawa 258-8577 (JP)**
- **HANDO, Rie**
**Ashigarakami-gun, Kanagawa 258-8577 (JP)**

(74) Representative: **Hoffmann Eitle**
**Patent- und Rechtsanwälte PartmbB**
**Arabellastraße 30**
**81925 München (DE)**

Remarks:
The complete document including Reference Table(s) and the Sequence Listing(s) can be downloaded from the EPO website

# (54) CELL PRODUCTION METHOD, CELLS, AND PROTEIN PRODUCTION METHOD

(57) A donor vector of a gene of interest is introduced into a host cell, a recombinase is caused to act, and a cell expressing the gene of interest is selected from the host cell. The genome of the host cell has a region R including, in this order, one each of RRS1, RRS2, RRS3, and RRS4, which are recognition sites of the recombinase, and the donor vector has RRS5 and RRS6, which are recognition sites of the recombinase, and the gene of interest disposed between RRS5 and RRS6. RRS1 and RRS4 are recombinable with RRS5 and not recombinable with RRS6, and RRS2 and RRS3 are recombinable with RRS6 and not recombinable with RRS5.

FIG. 1

Region R
Host genome
RRS1
RRS2
RRS3
RRS4
1st MG
2nd MG
GoI
RRS5
RRS6
Donor vector
GoI
RRS5
RRS6
Donor vector
Region G
Host genome
Site1
Site2
Site3
Site4
GoI
GoI



Processed by Luminess, 75001 PARIS (FR)

## Description

## BACKGROUND OF THE INVENTION

1. Field of the Invention

The present disclosure relates to a method for producing a cell, a cell, and a method for producing a protein.

2. Description of the Related Art

EP2711428A discloses a site-specific integration host cell containing an endogenous Fer1L4 gene, in which an exogenous nucleotide sequence is integrated into the Fer1L4 gene.

WO2017/184831A discloses a cell comprising an exogenous nucleic acid integrated at a specific site in an expression-enhancing locus, in which the exogenous nucleic acid sequence encodes a bispecific antigen-binding protein.

WO2017/184832A discloses a cell comprising a first exogenous nucleic acid integrated into a first expression-enhancing locus and a second exogenous nucleic acid integrated into a second expression-enhancing locus, in which both the first and second exogenous nucleic acids encode an antigen-binding protein.

WO2020/072480A discloses a mammalian cell comprising a first recombinant target site (RTS) chromosomally integrated at a first high integration (HI) locus, in which the first HI locus is within about 30,000 base pairs of a genome compartment of active chromatin that is accessible and a TAD boundary, and the first HI locus overlaps with a region of a cell genome that interacts with at least one enhancer element.

Molecular Cell, 2003, Vol. 12, 1101-1111 discloses the directionality of DNA integration by Bxb1 integrase depends only on the central dinucleotide of attP and attB.

BMC Biotechnology, 2013, 13:87 discloses among 15 serine recombinase candidates for integrating DNA into the human genome, Bxb1 integrase is the most excellent in terms of accuracy and efficiency.

Acta Biochim Biophys Sin, 2017, 49(1), 44-50 discloses among four serine integrases φBT1, TG1, φRv1, and Bxb1, Bxb1 integrase is the most efficient.

## SUMMARY OF THE INVENTION

There is a technique for incorporating a gene of interest into a genome of a host cell for the purpose of creating a cell that stably produces a medical protein such as a humanized monoclonal antibody. From the viewpoint of cost, it is preferable that the type of the donor vector of the gene of interest and the type of the enzyme that recombines the donor vector and the host genome are small. In addition, from the viewpoint of the production amount of the target protein, it is preferable that a plurality of gene of interests are disposed in a high-expression region of the host genome.

For example, in a case where the target protein is an antibody, it is desirable that one type of donor vector carrying a gene of interest including a heavy chain coding sequence and a light chain coding sequence is introduced into a host cell, and a plurality of gene of interests are inserted into a high-expression region of the host genome by a recombination reaction of one type of recombinase.

The present disclosure has been made under the above circumstances.

An object of the present disclosure is to provide a method for producing a cell that highly expresses a gene of interest.

Another object of the present disclosure is to provide a cell that highly expresses a gene of interest.

Another object of the present disclosure is to provide a method for producing a protein having excellent productivity.

The specific means for achieving the objects includes the following aspects.

<1> A method for producing a cell by integrating a gene of interest into a genome of a host cell using one type of recombinase and one type of donor vector, the method comprising:

introducing the donor vector for the gene of interest into the host cell;
causing the recombinase to act in the host cell into which the donor vector has been introduced; and
selecting, from the host cell in which the recombinase has been caused to act, a cell expressing the gene of interest,
in which the genome of the host cell and the donor vector satisfy the following (1) to (4),

(1) the genome of the host cell has a region R including, in this order, one each of RRS1, RRS2, RRS3, and RRS4, which are recognition sites of the recombinase,

(2) the donor vector has RRS5 and RRS6, which are recognition sites of the recombinase, and the gene of interest disposed between RRS5 and RRS6,
(3) RRS1 and RRS4 are recombinable with RRS5 and not recombinable with RRS6, and
(4) RRS2 and RRS3 are recombinable with RRS6 and not recombinable with RRS5.

<2> The method for producing a cell according to <1>, in which the genome of the host cell further satisfies the following (5),
(5) RRS1 and RRS4 have an identical sequence, and RRS2 and RRS3 have an identical sequence.
<3> The method for producing a cell according to <1> or <2>, in which the donor vector further satisfies the following (6),
(6) a transcription direction of the gene of interest disposed between RRS5 and RRS6 is a direction from RRS6 toward RRS5.
<4> The method for producing a cell according to any one of <1> to <3>, in which the genome of the host cell further satisfies the following (7),
(7) the region R includes a first selectable marker gene disposed between RRS1 and RRS2 and a second selectable marker gene disposed between RRS3 and RRS4.
<5>
The method for producing a cell according to any one of <1> to <4>, in which the donor vector further satisfies the following (8),
(8) the donor vector includes a third selectable marker gene disposed between RRS5 and RRS6.
<6> The method for producing a cell according to any one of <1> to <5>, further comprising:
introducing an expression vector of the recombinase into the host cell.
<7> The method for producing a cell according to any one of <1> to <6>, in which the recombinase is a serine recombinase.
<8> The method for producing a cell according to any one of <1> to <7>, in which the host cell is a mammalian cell.
<9> The method for producing a cell according to any one of <1> to <7>, in which the host cell is a CHO cell.
<10> The method for producing a cell according to any one of <1> to <9>, in which the gene of interest is a gene encoding at least one selected from the group consisting of an enzyme, an antibody, an interleukin, a cytokine, a chemokine, a hormone, a growth factor, a transcription factor, a receptor, a viral preparation, a vaccine, a therapeutic protein, subunits thereof, and fragments thereof.
<11> A cell in which a gene of interest is integrated into a genome, in which the cell satisfies the following (A) to (C),

(A) the genome has a region G including one each of site 1, site 2, site 3, and site 4, in this order, the sites being sites formed by recombination of recognition sites of a recombinase,
(B) site 1 and site 4 have sequence identity, and site 2 and site 3 have sequence identity, and
(C) the region G includes the gene of interest disposed between site 1 and site 2 and the gene of interest disposed between site 3 and site 4.

<12> The cell according to <11>, in which the cell further satisfies the following (D),
(D) a transcription direction of the gene of interest disposed between site 1 and site 2 is a direction from site 2 toward site 1, and a transcription direction of the gene of interest disposed between site 3 and site 4 is a direction from site 3 toward site 4.
<13> The cell according to <11> or <12>, in which the recombinase is a serine recombinase.
<14> The cell according to any one of <11> to <13>, in which the cell is a mammalian cell.
<15> The cell according to any one of <11> to <13>, in which the cell is a CHO cell.
<16> The cell according to any one of <11> to <15>, in which the gene of interest is a gene encoding at least one selected from the group consisting of an enzyme, an antibody, an interleukin, a cytokine, a chemokine, a hormone, a growth factor, a transcription factor, a receptor, a viral preparation, a vaccine, a therapeutic protein, subunits thereof, and fragments thereof.
<17> A method for producing a protein, the method comprising:
culturing the cell according to any one of <11> to <16> to express a protein encoded by the gene of interest.

According to the present disclosure, there is provided a method for producing a cell in which a gene of interest is highly expressed.

According to the present disclosure, there is provided a cell in which a gene of interest is highly expressed.

According to the present disclosure, there is provided a method for producing a protein having excellent productivity.

## BRIEF DESCRIPTION OF THE DRAWINGS

FIG. 1 is a conceptual diagram showing a recombination form of a region R of a host genome and a donor vector.
FIG. 2 is a schematic configuration diagram of a vector for constructing a host genome used in Examples.
FIG. 3 is a schematic configuration diagram of a donor vector used in Examples.
FIG. 4 is a schematic configuration diagram of a recombinase expression vector used in Examples.
FIG. 5 is a schematic configuration diagram of a region G of a clone produced in Examples.

## DESCRIPTION OF THE PREFERRED EMBODIMENTS

Hereinafter, embodiments according to the present disclosure will be described. These descriptions and Examples are only illustrative of the embodiments and do not limit the scope of the embodiments. The action mechanism mentioned in the present disclosure includes estimation, and the accuracy thereof does not limit the scope of the embodiments.

In a case where the embodiments of the present disclosure are described with reference to the drawings, the configurations of the embodiments of the present disclosure are not limited to the configurations shown in the drawings. The sizes of the elements in the drawings are conceptual, and the relative relationship between the sizes of the elements is not limited thereto.

In the present disclosure, the term "step" includes not only an independent step, but also a step that may not be clearly distinguished from other steps but still achieves a desired effect of the step.

In the present disclosure, a numerical range expressed using "to" indicates a range including numerical values before and after "to" as a minimum value and a maximum value.

In a numerical range described in a stepwise manner in the present disclosure, an upper limit or a lower limit described in one numerical range may be replaced with an upper limit or a lower limit in another numerical range described in a stepwise manner. In addition, in the numerical ranges described in the present disclosure, the upper limit value or the lower limit value of the numerical range may be replaced with the value shown in Examples.

In the present disclosure, each component may contain a plurality of kinds of substances corresponding thereto. In the present disclosure, upon referring to an amount of each component in a composition, the amount means a total amount of a plurality of types of substances present in the composition unless otherwise specified, in a case where a plurality of types of substances corresponding to each component are present in the composition.

In the present disclosure, the nucleic acid is a term including any nucleic acid (for example, DNA, RNA, an analog thereof, a natural product, or an artificial product) and a nucleic acid in which a low-molecular-weight compound, a group (for example, a methyl group), a molecule other than a nucleic acid, a structure, or the like is linked to any nucleic acid. The nucleic acid may be single-stranded or double-stranded.

In the present disclosure, the donor vector is a substance having an action of introducing an exogenous nucleic acid into a cell and a genome of the cell, and is a nucleic acid itself. The origin, the form, and the base sequence of the donor vector are not limited. The donor vector may be a cyclic nucleic acid or a linear nucleic acid. The donor vector may be a single-stranded nucleic acid or a double-stranded nucleic acid. The donor vector is preferably a double-stranded DNA.

In the present disclosure, the number of amino acid residues of the protein is not limited. The protein includes a protein in which an amino acid is post-translationally modified. Examples of the post-translational modification of an amino acid include phosphorylation, methylation, acetylation, glycosylation, lipidation, and the like.

In the present disclosure, in the notation of an amino acid, the three-letter notation and the one-letter notation established by IUPAC-IUBMB joint commission on Biochemical Nomenclature (IUPAC-IUBMB JCBN) are used. Unless otherwise specified, the amino acid referred to in the present disclosure is an L-amino acid.

In the present disclosure, the identity of the base sequence and the identity of the amino acid sequence are calculated using a basic local alignment search tool (BLAST) (https://blast.ncbi.nlm.nih.gov/Blast.cgi).

In the present disclosure, the recombinase is a general term for an enzyme that recombines a nucleic acid, and is a term including an integrase. RRS is an abbreviation for a recombinase recognition site.

In the present disclosure, in a case of referring to the orientation or the base sequence of the recombinase recognition site (RRS), among two DNA strands constituting a double-stranded DNA, a DNA strand displaying a recognition sequence of a recombinase is referred to as a sense strand, and a complementary strand of the sense strand is referred to as an antisense strand.

In the present disclosure, the identity of the base sequence of the RRS means the identity of the base sequence read in a 5' $\rightarrow$ 3' direction of the sense strand (that is, the DNA strand displaying the recognition sequence of the recombinase).

<Method for Producing Cell>

The present disclosure provides a method for producing a cell in which a gene of interest is highly expressed.

The method for producing a cell according to the present disclosure is a method for producing a cell by incorporating a gene of interest into a genome of a host cell using one kind of recombinase and one kind of donor vector.

A method for producing a cell is

introducing the donor vector for the gene of interest into the host cell;
causing the recombinase to act in the host cell into which the donor vector has been introduced;
selecting, from the host cell in which the recombinase has been caused to act, a cell expressing the gene of interest.

The origin, the size, and the base sequence of the gene of interest are not limited.

Examples of the gene of interest include a gene encoding at least one selected from the group consisting of an enzyme, an antibody, interleukin, a cytokine, a chemokine, a hormone, a growth factor, a transcription factor, a receptor, a viral preparation, a vaccine, a therapeutic protein, a subunit thereof, and a fragment thereof.

That is, examples of a protein encoded by the gene of interest (referred to as a "target protein" in the present disclosure) include at least one selected from the group consisting of an enzyme, an antibody, interleukin, a cytokine, a chemokine, a hormone, a growth factor, a transcription factor, a receptor, a protein constituting a viral preparation, a vaccine, a therapeutic protein, a subunit thereof, and a fragment thereof.

In the present disclosure, the antibody is not limited to an immunoglobulin and may be any molecule that binds to an antigen. In the present disclosure, the antibody is a term includes an antibody fragment and an antigen-binding molecule. In the present disclosure, the heavy chain of the antibody is also referred to as an H chain, and the light chain of the antibody is also referred to as an L chain.

The gene of interest has all sequences necessary for the expression of the target protein. That is, the gene of interest includes a coding sequence of the target protein and all nucleic acids (for example, a promoter, a transcription terminator, and a polyadenylation sequence) necessary for transcription and translation of the coding sequence in a host cell. The gene of interest may include one copy of a coding sequence of a protein, or may include two or more copies thereof. For example, the gene of interest may include at least one copy of a coding sequence of each subunit in order to express all subunits of a heteromultimeric protein. For example, the gene of interest may have at least one copy of a sequence encoding an H chain of an antibody and at least one copy of a sequence encoding an L chain of the antibody.

The gene of interest may further include a sequence encoding at least one selected from the group consisting of a nucleic acid constituting a viral preparation, a transfer control nucleic acid, and a non-coding RNA. Examples of the non-coding RNA (ncRNA) include microRNA (miRNA), short hairpin RNA (shRNA), small interfering RNA (siRNA), small nuclear RNA (snRNA), ribosomal RNA (rRNA), and transfer RNA (tRNA).

The host cell may be a prokaryotic cell or a eukaryotic cell. Examples of the prokaryotic cell include a bacterial cell. Examples of the eukaryotic cell include fungi, yeast, insect cells, and mammalian cells.

Examples of the bacterial cell include Gram-negative bacterial cells such as Escherichia coli, Salmonella typhimurium, Serratia marcescens, Pseudomonas putida, and Pseudomonas aeruginosa; and Gram-positive bacterial cells such as Bacillus subtilis. Suitable bacterial cells are Enterobacteriaceae, and more suitable examples thereof include Escherichia coli, particularly B strain or K12 strain.

Examples of the fungi include Aspergillus oryzae.

Examples of the yeast include budding yeast (Saccharomyces cerevisiae), Pichia pastoris, and Hansenula polymorpha.

Examples of the insect cell include a BmN cell derived from silkworm (Bombyx mori), an Sf9 cell and an Sf21 cell derived from cabbage armyworm (Spodoptera frugiperda), an S2 cell derived from fruit fly (Drosophila melanogaster), and Pv11 cells derived from sleeping chironomid (Polypedilum vanderplanki).

Examples of the mammalian cell include Chinese hamster ovary cells (CHO cells), baby hamster kidney cells (BHK cells), a human embryonic kidney cell line (for example, HEK293 cells), a human retinoblast-derived cell line (for example, PER.C6 cells), a mouse myeloma cell line (for example, NS0 cells and SP2/0 cells), and established cell lines derived from these cells.

Examples of the CHO cell include a CHO-DG44 cell, a CHO-K1 cell, a CHO-DXB11 cell, a CHOpro3$^-$ cell, and an established cell derived from these cells.

Examples of the mammalian cell include cells having a differentiation ability into other cells. Examples thereof include pluripotent stem cells such as embryonic stem cells (ES cells) and induced pluripotent stem cells (iPS cells); multipotent stem cells such as mesenchymal stem cells, tissue stem cells, and somatic stem cells; and the like.

Examples of a means for introducing the expression vector into a host cell include electroporation, lipofection, microinjection, and cell infection with a viral vector. From the viewpoints of high safety, high introduction efficiency, and low cytotoxicity, electroporation is preferable.

The reaction of the recombinase in the host cell into which the donor vector has been introduced is realized, for example, by maintaining the culture environment of the host cell at the optimal temperature of the recombinase.

The recombinase may be an enzyme that is present in the host cell, an enzyme that is introduced into the host cell by an expression vector, or an enzyme that is added to the host cell in the form of a protein or RNA. The recombinase expression vector may be incorporated into the host genome or may be present in the host cell as an extrachromosomal element.

An example of an embodiment of the method for producing a cell according to the present disclosure includes introducing a recombinase expression vector into a host cell.

From the viewpoint of reliably acting inside the host cell at a desired timing, it is preferable that the recombinase is introduced into the host cell by an expression vector.

The order in which the recombinase expression vector and the donor vector of the gene of interest are introduced into the host cell is not limited. The recombinase expression vector and the donor vector of the gene of interest may be introduced into the host cell together or separately.

From the viewpoint of not increasing the number of steps and the time required for producing the target cell, it is preferable to introduce the recombinase expression vector and the donor vector of the gene of interest into the host cell together.

The base nucleic acid and base sequence for constructing the recombinase expression vector are not limited. Examples of the base nucleic acid include a viral vector, a non-viral vector, and an artificial nucleic acid. The base nucleic acid may be a cyclic nucleic acid or a linear nucleic acid.

Examples of the nucleic acid derived from a viral vector include a nucleic acid derived from an adenovirus, an adeno-associated virus, a retrovirus, a vaccinia virus, a poxvirus, a lentivirus, a herpes virus, a baculovirus, or a bacteriophage.

Examples of the non-viral vector include an artificial plasmid and a bacterial vector in which a bacterial gene has been modified.

The origin, type, and form of the recombinase are not limited.

Examples of the recombinase that is widely used in genetic engineering include a serine recombinase (type having a serine residue in the active site) and a tyrosine recombinase (type having a tyrosine residue in the active site), which are enzymes derived from bacteriophages. These enzymes were found as enzymes that incorporate a phage genome into a bacterial genome when bacteriophages infect bacteria. It has been confirmed that some of the serine recombinases and the tyrosine recombinases function in mammalian cells.

Preferred characteristics of the recombinase used in the method for producing a cell according to the present disclosure include that the base sequence of the recognition site has high specificity, that the recombination reaction does not require factors other than the recombinase, and that the recombination reaction is irreversible.

From the viewpoint of having all of the above-described characteristics, a serine recombinase is preferable as the recombinase used in the method for producing a cell according to the present disclosure. From the viewpoint of being able to recombine a mammalian genome, the serine recombinase is preferably one selected from the group consisting of Bxb1, φC31, TP901, A118, SPβc, TG1, φBT1, φRv1, φ370.1, Wβ, Pa01, and Pa03. Among these, from the viewpoint of excellent accuracy and efficiency of the recombination reaction, a Bxb1 recombinase (also known as Bxb1 integrase) is preferable.

In a case of constructing an expression vector for a recombinase derived from a bacteriophage, a codon of a recombinase gene is optimized to a codon that can be expressed in a host cell. It is preferable that a coding sequence of a nuclear localization signal is added to the recombinase gene.

The selection of cells expressing the gene of interest from the host cells in which the recombinase has reacted is performed, for example, based on the concentration and/or purity of the target protein. For example, the selection is carried out by setting a reference value for the concentration and/or purity of the target protein and selecting cells that have reached the reference value; and selecting cells in which the concentration and/or purity of the target protein is relatively high. Specifically, the following (S1) to (S4) are carried out.

(S1) A selection drug is added to a culture medium of the host cell.
(S2) The host cell is subjected to single cell isolation.
(S3) A part of a culture solution of the single-celled cells is collected, and the concentration and/or purity of the target protein is measured.
(S4) Cells in which the concentration and/or purity of the target protein is equal to or higher than the reference value or cells in which the concentration and/or purity of the target protein is relatively high are selected.

The purity of the target protein means a proportion (mass basis or number basis) of the target protein in the total amount of a plurality of types of proteins. In a case where the target protein is a multimeric protein, a protein that is not in the original shape (for example, a protein in which a part of the subunit is missing or a protein in which a certain subunit is replaced with another subunit) may be generated, and it is desirable that the proportion of the protein that is not in the

original shape is low, that is, the purity of the protein in the original shape is high.

The genome of the host cell (also referred to as a "host genome" in the present disclosure) and the donor vector, which are used in the method for producing a cell according to the present disclosure, have the following forms (1) to (4).

(1) The genome of the host cell has a region R including one RRS1, one RRS2, one RRS3, and one RRS4, which are the recognition sites of the recombinase, in this order.
(2) The donor vector has RRS5 and RRS6, which are the recognition sites of the recombinase, and a gene of interest disposed between RRS5 and RRS6.
(3) RRS1 and RRS4 are recombination-competent with RRS5 and recombination-incompetent with RRS6.
(4) RRS2 and RRS3 are recombination-competent with RRS6 and recombination-incompetent with RRS5.

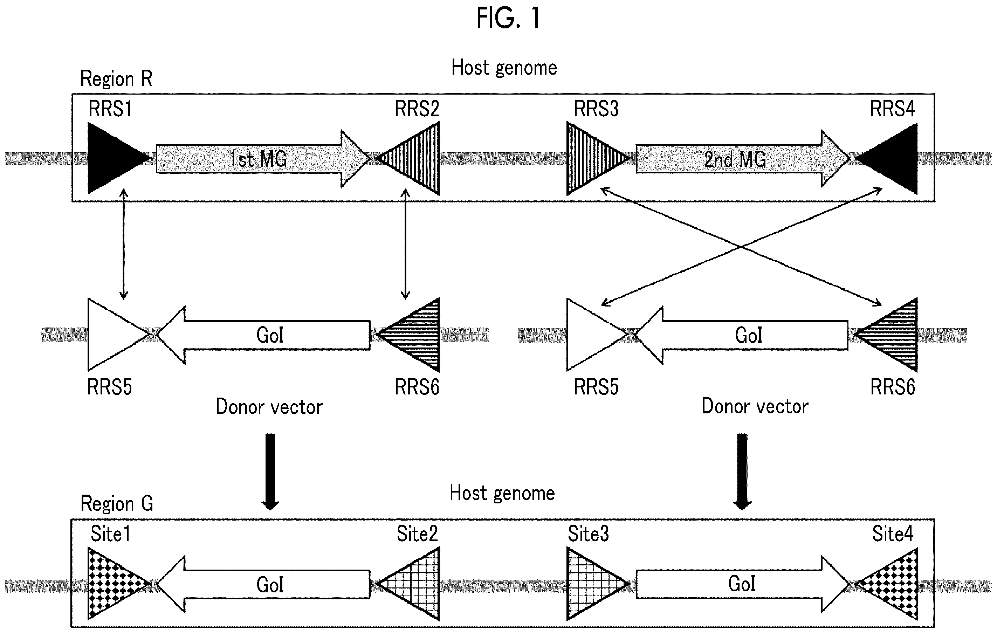

FIG. 1 shows a recombination form of the region R of the host genome and the donor vector. FIG. 1 shows a form in which the region R has a selectable marker gene, but the region R may not have a selectable marker gene. Abbreviations in FIG. 1 have the following meanings.

· GoI: gene of interest
· 1st MG: first selectable marker gene
· 2nd MG: second selectable marker gene

In the method for producing a cell according to the present disclosure, two gene of interests are incorporated into the region R of the host genome by using the host genome and the donor vector, which have the forms (1) to (4). By providing the region R in the high-expression region of the host genome, it is possible to efficiently incorporate two gene of interests into the high-expression region.

An example of the embodiment of the genome of the host cell is further the following form (5).

(5) RRS1 and RRS4 have the same sequence, and RRS2 and RRS3 have the same sequence.

In a case where the host genome has the form (5), the probability of realizing the incorporation of two gene of interests into the region R of the host genome is increased.

An example of the embodiment of the donor vector is further the following form (6).

(6) The transcription direction of the gene of interest disposed between RRS5 and RRS6 is a direction from RRS6 to RRS5.

The transcription direction of the gene of interest is a potential transcription direction in the donor vector. The form (6) means that the elements constituting the gene of interest, that is, the coding sequence of the target protein and all the nucleic acids (for example, a promoter, a transcription terminator, and a polyadenylation sequence) necessary for the transcription and translation of the coding sequence in the host cell are arranged from RRS6 to RRS5 in an order and orientation in which the transcription and translation are possible.

In a case where the donor vector has the form (6), the transcription directions of the two gene of interests arranged close to each other on the host genome are in directions (←→) away from each other as a result of recombination. In the form in which the transcription directions of the two adjacent gene of interests are in directions (←→) away from each other, the high expression of the gene of interest can be expected as compared with the form in which the transcription directions of the two adjacent gene of interests are in directions (→←) approaching each other.

The donor vector shown in FIG. 1 has the form (6). In the form after recombination shown in FIG. 1, the transcription directions of the two gene of interests arranged close to each other on the host genome are in directions (←→) away from each other.

An example of the embodiment of the genome of the host cell is further the following form (7).

(7) The region R has a first selectable marker gene disposed between RRS1 and RRS2 and a second selectable marker gene disposed between RRS3 and RRS4.

In a case where the host genome has the form (7), it is easy to select and concentrate the host cell in which recombination has occurred inside the region R after the recombinase is reacted, or it is easy to select and concentrate the host cell in which the region R is present in the genome before the recombinase is reacted.

The region R of the host genome shown in FIG. 1 has the form (7). The transcription direction of the first selectable marker gene and the transcription direction of the second selectable marker gene may be any of the same direction (→→ or ←←), a direction in which the transcription directions approach each other (→←), or a direction in which the transcription directions are away from each other (←→).

An example of the embodiment of the donor vector is further the following form (8).

(8) The donor vector has a third selectable marker gene disposed between RRS5 and RRS6.

In a case where the donor vector has the form (8), it is easy to select and concentrate the host cell in which the gene of interest is incorporated into the genome.

Hereinafter, RRS1 to RRS6, the genome of the host cell, and the donor vector will be described in detail.

[RRS 1 to RRS6]

First, the characteristics of the RRS of the serine recombinase will be described.

The RRS of the serine recombinase is generally called attP (phage attachment site) and attB (bacterial attachment site) in consideration of the fact that the serine recombinase is derived from a bacteriophage. The serine recombinase recombines DNA between attP and attB.

A base sequence similar to the native attP or attB, and a sequence recognized by the serine recombinase are called pseudo attP and pseudo attB.

The number of bases of attP and pseudo attP may be in a range of 1 bp to 1,000 bp, and is generally in a range of 10 bp to 300 bp and more generally in a range of 20 bp to 200 bp.

The number of bases of attB and pseudo attB may be in a range of 1 bp to 1,000 bp, and is generally in a range of 10 bp to 300 bp and more generally in a range of 20 bp to 200 bp.

Hereinafter, as an example of the RRS of the serine recombinase, an example of a native attP and a native attB of a Bxb1 recombinase (also known as a Bxb1 integrase), and a pseudo attP and a pseudo attB will be shown.

The RRS of the serine recombinase may determine whether or not attP and attB can be recombined depending on the difference between two bases at the center of the sequence or in the vicinity thereof (referred to as "central portion" in the present disclosure). The Bxb 1 recombinase usually determines whether or not attP and attB can be recombined depending on the difference between two bases in the central portion. In each of the following sequences, two bases in the central portion related to whether or not attP and attB can be recombined are underlined.

Native attP

SEQ ID NO1: 5'-GTCGTGGTTTGTCTGGTCAACCACCGCG GT CTCAGTGGTGTACGGTACAAACCCCGAC-3'

Native attB

SEQ ID NO2: 5'-TCGGCCGGCTTGTCGACGACGGCG GT CTCCGTCGTCAGGATCATCCGGGC-3'

Example of Pseudo attP

SEQ ID NO3: 5'-GTCGTGGTTTGTCTGGTCAACCACCGCG CT CTCAGTGGTGTACGGTACAAACCCCGAC-3'

Example of Pseudo attB

SEQ ID NO4: 5'-TCGGCCGGCTTGTCGACGACGGCG CT CTCCGTCGTCAGGATCATCCGGGC-3'

SEQ ID NO: 3 is a sequence in which the first base of the central portion "GT" of SEQ ID NO: 1 is modified to change the central portion to "CT".

SEQ ID NO: 4 is a sequence in which the first base of the central portion "GT" of SEQ ID NO: 2 is modified to change the central portion to "CT".

In the RRS of the serine recombinase, whether or not recombination between attP and attB is possible may be determined by the difference or similarity between two bases of the central portion. In the case of SEQ ID NO: 1 to SEQ ID NO: 4, whether or not recombination is possible is as follows.

SEQ ID NO: 1 and SEQ ID NO: 2, in which the two bases of the central portion are the same, are recombination-competent. SEQ ID NO: 3 and SEQ ID NO: 4, in which the two bases of the central portion are the same, are recombination-competent.

SEQ ID NO: 1 and SEQ ID NO: 4, in which the two bases of the central portion are different, are non-recombinogenic. SEQ ID NO: 3 and SEQ ID NO: 2, in which the two bases of the central portion are different, are non-recombinogenic.

In the present disclosure, the term "non-recombinogenic" includes a form in which recombination is not possible and a form in which the probability of recombination occurring is lower than the expected value.

It is preferable that RRS1 to RRS6 are designed as base sequences recognized by the same type of serine recombinase in consideration of the two-base sequence of the central portion. Specifically, a form including the following forms (a) to (h) is preferable. Forms (3) and (4) are easily realized by forms (a) to (h).

(a) RRS1 and RRS4 have the same two bases in the central portion and have the same base sequence as a whole. The identity of the entire base sequence is preferably 80% or more, more preferably 90% or more, still more preferably 95% or more, and most preferably 100%.
(b) RRS2 and RRS3 have the same two bases in the central portion and have the same base sequence as a whole. The identity of the entire base sequence is preferably 80% or more, more preferably 90% or more, still more preferably 95% or more, and most preferably 100%.
(c) RRS1 (and RRS4) and RRS2 (and RRS3) have one or both of the two bases in the central portion different from each other and have the same base sequence as a whole. The identity of the entire base sequence is preferably 80% or more, more preferably 90% or more, and still more preferably 95% or more. It is preferable that RRS1 (and RRS4) and RRS2 (and RRS3) have the same sequence except for one or both of the two bases in the central portion.
(d) The number of bases in each of RRS1 to RRS4 is preferably 1 bp to 1,000 bp, more preferably 10 bp to 300 bp, and still more preferably 20 bp to 200 bp. The difference in the number of bases in RRS1 to RRS4 is preferably 30% or less, more preferably 20% or less, and still more preferably 15% or less. It is most preferable that the number of bases in RRS 1 to RRS4 is the same.
(e) The two bases in the central portion of RRS5 are the same as the two bases in the central portion of RRS1 and RRS4.
(f) The two bases in the central portion of RRS6 are the same as the two bases in the central portion of RRS2 and RRS3.
(g) RRS5 and RRS6 have one or both of the two bases in the central portion different from each other and have the same base sequence as a whole. The identity of the entire base sequence is preferably 80% or more, more preferably 90% or more, and still more preferably 95% or more. It is preferable that RRS5 and RRS6 have the same sequence except for one or both of the two bases in the central portion.
(h) The number of bases in each of RRS5 and RRS6 is preferably 1 bp to 1,000 bp, more preferably 10 bp to 300 bp, and still more preferably 20 bp to 200 bp. The difference in the number of bases in RRS5 and RRS6 is preferably 30% or less, more preferably 20% or less, and still more preferably 15% or less. It is most preferable that the number of bases in RRS5 and RRS6 is the same.
In a case where a serine recombinase is used for producing a cell, examples of the embodiments of RRS1 to RRS6 include the following forms (i) and (j).
(i) RRS1 to RRS4 are a native attP and a pseudo attP of a serine recombinase, and RRS5 and RRS6 are a native attB and a pseudo attB of a serine recombinase. RRS1, RRS4, and RRS5 (or RRS2, RRS3, and RRS6) are a native att of a serine recombinase.
(j) RRS1 to RRS4 are a native attB and a pseudo attB of a serine recombinase, and RRS5 and RRS6 are a native attP and a pseudo attP of a serine recombinase. RRS1, RRS4, and RRS5 (or RRS2, RRS3, and RRS6) are a native att of a serine recombinase.

The native recognition sequence of the serine recombinase can be known from academic papers, technical documents, and the like.
There are 16 sequences applicable to the two bases of the central portion of attP and attB. That is, in the 5' $\rightarrow$ 3' direction, the sequences are "GT", "CT", "AT", "TT", "GA", "CA", "AA", "TA", "GC", "CC", "AC", "TC", "GG", "CG", "AG", and "TG". Among these, two bases of the central portion of the pseudo att are selected.
In a case where the Bxb1 recombinase is used for the production of cells, examples of the embodiments of RRS1 to RRS6 include the following. The present embodiment is referred to as Ex (1).

RRS1 and RRS4 are SEQ ID NO: 1,
RRS2 and RRS3 are SEQ ID NO: 3,
RRS5 is SEQ ID NO:5, and
RRS6 is SEQ ID NO:4.

In a case where the Bxb1 recombinase is used for the production of cells, examples of the embodiments of RRS1 to RRS6 include the following. The present embodiment is referred to as Ex (2).

RRS1 and RRS4 are SEQ ID NO: 4,
RRS2 and RRS3 are SEQ ID NO: 1,
RRS5 is SEQ ID NO:4, and

RRS6 is SEQ ID NO:2.

In a case where the Bxb1 recombinase is used for the production of cells, examples of the embodiments of RRS1 to RRS6 include the following. The present embodiment is referred to as Ex (3).

RRS1 and RRS4 are SEQ ID NO: 2,
RRS2 and RRS3 are SEQ ID NO: 4,
RRS5 is SEQ ID NO: 1, and
RRS6 is SEQ ID NO:3.

In a case where the Bxb1 recombinase is used for the production of cells, examples of the embodiments of RRS1 to RRS6 include the following. The present embodiment is referred to as Ex (4).

RRS1 and RRS4 are SEQ ID NO: 4,
RRS2 and RRS3 are SEQ ID NO: 2,
RRS5 is SEQ ID NO:3, and
RRS6 is SEQ ID NO:1.

Examples of another embodiment of RRS1 to RRS6 include a form in which two bases of the central portion are modified based on Ex (1) to Ex (4). Two of the 16 two-base sequences are selected, one is assigned to the two bases of the central portion of RRS1, RRS4, and RRS5, and the other is assigned to the two bases of the central portion of RRS2, RRS3, and RRS6.

Table 1 shows an example of the embodiment of Ex (1). Table 1 shows only the bases of the sense strand (the DNA strand on the side displaying the recombinase recognition sequence), and the bases of the antisense strand are omitted. The direction of the arrow is the 5' → 3' direction of the sense strand. In each sequence shown in Table 1, the two bases of the central portion related to the possibility of recombination between RRSs are underlined.

[Table 1]

RRS1 (Sequence Number_1)

5' GTCGTGGTTT GTCTGGTCAA CCACCGCGGT CTCAGTGGTG TACGGTACAA ACCCCGAC 3'

3' ---------- ---------- ---------- --------- ---------- --------- 5'

RRS2 (Sequence Number_3)

5' ---------- ---------- ---------- --------- ---------- --------- 3'

3' CAGCCCCA AACATGGCAT GTGGTGACTC TCGCGCCACC AACTGGTCTG TTTGGTGCTG 5'

RRS3 (Sequence Number_3)

5' GTCGTGGTTT GTCTGGTCAA CCACCGCGCT CTCAGTGGTG TACGGTACAA ACCCCGAC 3'

3' ---------- ---------- ---------- --------- ---------- --------- 5'

RRS4 (Sequence Number_1)

5' ---------- ---------- ---------- --------- ---------- --------- 3'

3' CAGCCCCA AACATGGCAT GTGGTGACTC TGGCGCCACC AACTGGTCTG TTTGGTGCTG 5'

RRS5 (Sequence Number_2)

5' TCGGCCGGCT TGTCGACGAC GGCGGTCTCC GTCGTCAGGA TCATCCGGGC 3'

3' ---------- ---------- ---------- --------- ---------- 5'

5' Gene of interest 3'

3' --------------- 5'

RRS6 (Sequence Number_4)

5' ---------- ---------- ---------- --------- ---------- 3'

3' CGGGCCTACT AGGACTGCTG CCTCTCGCGG CACGAGCTGT TCGGCCGGCT 5'

Ex (1) has another form in addition to the form shown in Table 1, in the orientation of each RRS. The orientation of each RRS is not limited to the form shown in Table 1 as long as the orientation realizes the movement of the gene of interest from the donor vector to two sites in the region R.

In a case where the serine recombinase is used for the production of cells, it is preferable that the orientations of RRS1 to RRS4 in the region R and the orientations of RRS5 and RRS6 in the donor vector are the following forms in order to efficiently realize the movement of the gene of interest from the donor vector to two sites in the region R.

In the following description, the orientation of RRS is shown in the 5' → 3' direction of the sense strand (the DNA

strand on the side displaying the recombinase recognition sequence).

· In a case where the orientations of RRS5 and RRS6 are "→ gene of interest ←", the orientations of RRS1, RRS2, RRS3, and RRS4 are "→ ← → ←". (Form shown in Table 1)
· In a case where the orientations of RRS5 and RRS6 are "← gene of interest →", the orientations of RRS1, RRS2, RRS3, and RRS4 are "← → ← →".
· In a case where the orientations of RRS5 and RRS6 are "→ gene of interest →", the orientations of RRS1, RRS2, RRS3, and RRS4 are "→ → ← ←"
· In a case where the orientations of RRS5 and RRS6 are "← gene of interest ←", the orientations of RRS1, RRS2, RRS3, and RRS4 are "← ← → →".

The orientations of RRS1 and RRS4 are opposite to each other. That is, the sense strand of RRS1 and the sense strand of RRS4 are different DNA strands.
The orientations of RRS2 and RRS3 are opposite to each other. That is, the sense strand of RRS2 and the sense strand of RRS3 are different DNA strands.

[Genome of host cell]

The genome of the host cell (also referred to as a "host genome" in the present disclosure) has the region R. The region R is a region including one RRS1, one RRS2, one RRS3, and one RRS4, which are recombinase recognition sites. The order of the RRSs in the region R is RRS1, RRS2, RRS3, and RRS4.
The region R is a continuous region. The host genome may have one region R or two or more regions R in the entire genome.
In the region R, a gene of interest can be inserted between RRS1 and RRS2 and a gene of interest can be inserted between RRS3 and RRS4 by recombination with the donor vector. Therefore, two gene of interests can be inserted per region.
An example of the embodiment of the host genome has, in the region R, a first selectable marker gene disposed between RRS1 and RRS2 and a second selectable marker gene disposed between RRS3 and RRS4.
The first selectable marker gene and the second selectable marker gene each include all nucleic acids necessary for gene expression. The size and base sequence of the first selectable marker gene and the second selectable marker gene are not limited.
The first selectable marker gene and the second selectable marker gene may be the same gene or different genes. From the viewpoint of not increasing the number of steps and time required for the selection and concentration of cells, it is preferable that the first selectable marker gene and the second selectable marker gene are the same gene.
An example of the embodiment of the first selectable marker gene and the second selectable marker gene is a negative selection gene used for the selection and concentration of host cells in which recombination has occurred inside the region R.
Examples of the negative selection gene include a suicide gene that induces cell death by a specific drug. Examples of the suicide gene include a herpes simplex virus-derived thymidine kinase gene (selection drug: ganciclovir), an inducible caspase 9 gene (selection drug: AP1903), and a cytosine deaminase gene (selection drug: 5-fluorocytosine).
An example of the embodiment of the first selectable marker gene and the second selectable marker gene is a gene that expresses a positive selection marker used for the selection and concentration of host cells in which the region R is present in the genome.
Examples of the positive selection marker include a fluorescent protein. Any known fluorescent protein can be used as the fluorescent protein. The fluorescent protein is preferably a monomer type high-brightness fluorescent protein.
In an example of the embodiment, one of the negative selection gene and the positive selection gene is disposed between the RRS1 and the RRS2. In another example of the embodiment, both the negative selection gene and the positive selection gene are disposed between the RRS1 and the RRS2.
In an example of the embodiment, one of the negative selection gene and the positive selection gene is disposed between the RRS3 and the RRS4. In another example of the embodiment, both the negative selection gene and the positive selection gene are disposed between the RRS3 and the RRS4.
In the region R, the number of bases between the outer end of the RRS1 and the outer end of the RRS4, which is the recognition site farthest from the RRS1, is, for example, 100 kbp or less, 70 kbp or less, 50 kbp or less, 30 kbp or less, or 10 kbp or less.
The number of bases between the outer end of the RRS1 and the outer end of the RRS4 is, for example, 100 bp or more, 1 kbp or more, or 2 kbp or more.
In the region R, the number of bases between the outer end (end close to the RRS1) of the RRS2 and the outer end (end close to the RRS4) of the RRS3 is preferably 50 bp or more, more preferably 100 bp or more, and still more

preferably 200 bp or more. According to the present embodiment, the two gene of interests to be inserted into the region R by recombination are arranged in close proximity to each other at an appropriate distance, and high expression of the gene of interest can be expected.

The region R may be a region that is already present in the host genome or may be a region that is newly formed in the host genome.

The formation of the region R in the host genome is carried out, for example, by incorporating the region R into the host genome using a vector equipped with the region R (referred to as a "vector for constructing a host genome" in the present disclosure).

The vector for constructing a host genome has at least RRS1, RRS2, RRS3, and RRS4 in this order. An example of the embodiment of the vector for constructing a host genome has a first selectable marker gene disposed between the RRS1 and the RRS2, and a second selectable marker gene disposed between the RRS3 and the RRS4.

The base nucleic acid and base sequence for constructing the vector for constructing a host genome are not limited. Examples of the base nucleic acid include a viral vector, a non-viral vector, and an artificial nucleic acid. The base nucleic acid may be a cyclic nucleic acid or a linear nucleic acid.

Examples of the nucleic acid derived from a viral vector include a nucleic acid derived from an adenovirus, an adeno-associated virus, a retrovirus, a vaccinia virus, a poxvirus, a lentivirus, a herpes virus, a baculovirus, or a bacteriophage.

Examples of the non-viral vector include an artificial plasmid and a bacterial vector in which a bacterial gene has been modified.

Examples of the embodiment of the host genome include a host genome in which the region R is inserted into at least one safe harbor in the host genome and the region R is present in the safe harbor.

The safe harbor in the genome is a region in which a host cell survives even in a case where a gene is inserted, and is a region in which the inserted gene is expressed. The safe harbor in the genome is specified by a chromosome number or an accession number and a base number of a public base sequence database. Examples of the public base sequence database include the International Nucleotide Sequence Databases (INSD), RefSeq (NCBI Reference Sequence Database), and the like.

The safe harbor in the genome may be referred to by the name of a known gene present in the region or in the vicinity of the region.

The safe harbor in the genome into which the region R is inserted may be a known safe harbor or a newly found safe harbor. The known safe harbor can be known from a publicly available database, an academic paper, a technical literature, and the like.

In a case where there are a large number of safe harbors, at least one safe harbor may be selected as the insertion region of the region R. Examples of a method of selecting the safe harbor include selecting a safe harbor in which the expression level (pg/cell/copy) of a protein encoded by the inserted gene is relatively high; and selecting a safe harbor in which the expression level (pg/cell/copy) of a protein encoded by the inserted gene exceeds a predetermined criterion. The protein expression level of the safe harbor may be data acquired from a publicly available database, an academic paper, a technical literature, and the like, or may be data obtained by actually inserting a gene into the safe harbor and measuring the protein expression level.

The insertion of the region R into the safe harbor in the genome can be performed by a known genome editing technology.

[Donor vector]

The donor vector has RRS5 and RRS6, which are recombinase recognition sites, and a gene of interest disposed between RRS5 and RRS6.

The gene of interest has all sequences necessary for the expression of the target protein. That is, the gene of interest includes a coding sequence of the target protein and all nucleic acids (for example, a promoter, a transcription terminator, and a polyadenylation sequence) necessary for transcription and translation of the coding sequence in a host cell. The gene of interest may include one copy of a coding sequence of a protein, or may include two or more copies thereof. For example, the gene of interest may include at least one copy of a coding sequence of each subunit in order to express all subunits of a heteromultimeric protein. For example, the gene of interest may have at least one copy of a sequence encoding an H chain of an antibody and at least one copy of a sequence encoding an L chain of the antibody.

The promoters that can be used in a prokaryotic cell include a promoter disclosed in J. Mol. Biol. 1986; 189(1): 113-30, a phage polymerase promoter, and an Escherichia coli polymerase promoter. The promoters that can be used in a prokaryotic cell include a promoter disclosed in J. Mol. Biol. 1986; 189(1): 113-30, a phage polymerase promoter, and an Escherichia coli polymerase promoter. Specific examples thereof include T7A1, T7A2, T7A3, λpL, λpR, lac, lacUV5, trp, tac, trc, phoA, and rrnB.

Examples of the promoters that can be used for yeast cells include a gal promoter, an AOX1 promoter, an AOX2

promoter, a GAP promoter, a GAL1 promoter, and a GAL10 promoter.

Examples of the promoters that can be used in insect cells include a polyhedrin promoter, a P10 promoter, an immediate early protein (IE-1) promoter of virus infection, an MT promoter, a COPIA promoter, a CMV promoter, an RSV promoter, an SV40 promoter, a heat shock protein promoter, an OPIE2 promoter, and an actin 5C promoter.

Examples of the promoters that can be used for mammalian cells include virus-derived promoters and housekeeping gene-derived promoters. Examples of the virus-derived promoters include a human CMV promoter, a rat CMV promoter, an SV40 promoter, an RSR-LTR promoter, and an HSK-TK promoter. Examples of the housekeeping gene-derived promoters include an hEF-1α promoter, a Chinese hamster EF-1α promoter, a β-actin promoter, and a mouse phosphoglycerate kinase (mPGK) promoter. A preferred example of the promoter that can be used for mammalian cells is an EF-1α promoter, and a more preferred example thereof is the hEF-1α promoter.

The gene of interest may include a coding sequence of a secretory leader for the intended purpose of promoting the transport or secretion of the target protein to the extracellular space. The secretion leader is one type of signal peptide, and is a signal peptide that induces transport or secretion of a polypeptide to the outside of a cell.

In a case where the gene of interest includes the coding sequence of the secretory leader, the coding sequence of the secretory leader and the coding sequence of the target protein are arranged in the same reading frame. Here, the "arranged in the same reading frame" means that both coding sequences are arranged to be capable of being expressed as one polypeptide of the secretory leader and the target protein. In the gene of interest, a coding sequence of a linker or a spacer may or may not be present between the coding sequence of the secretory leader and the coding sequence of the target protein.

A preferred form is a form in which the coding sequence of the target protein is arranged in the same reading frame downstream of the coding sequence of the secretory leader. A fusion protein in which the secretory leader is arranged on the N-terminal side of the target protein is expressed from the gene of interest of the present embodiment. A more preferred form is a form in which the coding sequence of the target protein is continuously arranged in the same reading frame downstream of the coding sequence of the secretory leader. A fusion protein in which the secretory leader is bonded to the N-terminal of the target protein is expressed from the gene of interest of the present embodiment. Here, the term "downstream" means the arrangement order of two coding sequences, and in a case where both coding sequences are arranged such that the coding sequence B is transcribed after the transcription of the coding sequence A, the coding sequence B is said to be arranged downstream of the coding sequence A.

The secretory leader of the fusion protein is generally cleaved from the fusion protein in the process of transporting or secreting the fusion protein.

Examples of the secretion leader include a fibronectin secretion leader, a collagen secretion leader, and an albumin secretion leader. From the viewpoint of a high secretion rate of the fusion protein to the extracellular space, the fibronectin secretion leader is preferable.

Examples of the fibronectin secretion leader include a fibronectin secretion leader of an amphibian and a fibronectin secretion leader of a mammal. A fibronection secretion leader of Xenopus laevis is given as an example of a fibronection secretion leader of an amphibian. Examples of the fibronectin secretion leader of a mammal include fibronectin secretion leaders of human, rat, mouse, cow, pig, dog, cat, and Chinese hamster, and functional equivalents thereof.

It is preferable to select an originating organism of the fibronectin secretion leader according to the type of the host cell. In a case where the host cell is a human cell, it is preferable to use a human fibronectin secretion leader as the gene of interest. In a case where the host cell is a rat cell, it is preferable to use a rat fibronectin secretion leader as the gene of interest. In a case where the host cell is a CHO cell, it is preferable to use a Chinese hamster fibronectin secretion leader as the gene of interest.

An example of the embodiment of the gene of interest includes an hEF-1α promoter, a coding sequence of a fibronectin secretion leader, a coding sequence of a target protein, and a polyA sequence, which are operatively linked to each other.

It is preferable that a transcription direction of the gene of interest disposed between RRS5 and RRS6 is a direction from RRS6 to RRS5. According to the present embodiment, transcription directions of two gene of interests inserted into the region R of the host genome are directions (←→) away from each other.

An example of the embodiment of the donor vector has a third selectable marker gene disposed between RRS5 and RRS6.

The third selectable marker gene includes all nucleic acids necessary for gene expression. A size and a base sequence of the third selectable marker gene are not limited.

The third selectable marker gene is a gene that expresses a positive selection marker used for selection and concentration of a host cell in which the gene of interest is incorporated into the genome.

Examples of the third selectable marker gene include a gene that exhibits resistance to a selection drug. Examples of the selection drug include an antibiotic and an enzyme inhibitor.

In a case where the selection drug is an antibiotic, an antibiotic resistance gene, which is a gene of an enzyme that

decomposes the antibiotic, is the selectable marker gene. Examples thereof include a hygromycin resistance gene, a neomycin resistance gene, a puromycin resistance gene, a chloramphenicol resistance gene, a tetracycline resistance gene, an erythromycin resistance gene, a spectinomycin resistance gene, a kanamycin resistance gene, a G418 resistance gene, a bleomycin resistance gene, a zeocin resistance gene, a phleomycin resistance gene, and an ampicillin resistance gene.

A DHFR-MTX system is given as an example in which the selective agent is an enzyme inhibitor. In the DHFR-MTX system, the selection drug is methotrexate (MTX), and the selectable marker gene is a dihydrofolate reductase (DHFR) gene. The DHFR-MTX system is an effective system in host cells (for example, CHO-DG44 cells) that are deficient in the DHFR gene.

A GS-MSX system is given as an example in which the selective agent is an enzyme inhibitor. In the GS-MSX system, the selection drug is methionine sulfoximine (MSX), and the selectable marker gene is a glutamine synthetase (GS) gene. The GS-MSX system is an effective system in a host cell (for example, a GS knockout CHO cell) that is deficient in the GS gene.

Examples of the third selectable marker gene include a gene of a fluorescent protein. Any known fluorescent protein can be used as the fluorescent protein. The fluorescent protein is preferably a monomer type high-brightness fluorescent protein.

In a case where the host genome has a gene of a fluorescent protein, it is preferable to avoid overlap between the excitation wavelength and the fluorescence wavelength between the fluorescent proteins.

As the third selectable marker gene, a plurality of the above-described genes may be used in combination. For example, a drug resistance gene and a gene of a fluorescent protein may be disposed between RRS5 and RRS6.

The base nucleic acid and base sequence for constructing the donor vector are not limited. Examples of the base nucleic acid include a viral vector, a non-viral vector, and an artificial nucleic acid. The base nucleic acid may be a cyclic nucleic acid or a linear nucleic acid.

Examples of the nucleic acid derived from a viral vector include a nucleic acid derived from an adenovirus, an adeno-associated virus, a retrovirus, a vaccinia virus, a poxvirus, a lentivirus, a herpes virus, a baculovirus, or a bacteriophage.

Examples of the non-viral vector include an artificial plasmid and a bacterial vector in which a bacterial gene has been modified.

<Cell>

The present disclosure provides a cell in which a gene of interest is highly expressed.

The cell of the present disclosure is a cell in which an exogenous gene of interest is incorporated into a genome.

The origin, the size, and the base sequence of the gene of interest are not limited.

Examples of the gene of interest include a gene encoding at least one selected from the group consisting of an enzyme, an antibody, interleukin, a cytokine, a chemokine, a hormone, a growth factor, a transcription factor, a receptor, a viral preparation, a vaccine, a therapeutic protein, a subunit thereof, and a fragment thereof.

That is, examples of the target protein include at least one selected from the group consisting of an enzyme, an antibody, an interleukin, a cytokine, a chemokine, a hormone, a growth factor, a transcription factor, a receptor, a protein constituting a viral preparation, a vaccine, a therapeutic protein, a subunit thereof, and a fragment thereof.

The gene of interest has all sequences necessary for the expression of the target protein. That is, the gene of interest includes a coding sequence of the target protein and all nucleic acids (for example, a promoter, a transcription terminator, and a polyadenylation sequence) necessary for transcription and translation of the coding sequence in the cell. The gene of interest may include one copy of a coding sequence of a protein, or may include two or more copies thereof. For example, the gene of interest may include at least one copy of a coding sequence of each subunit in order to express all subunits of a heteromultimeric protein. For example, the gene of interest may have at least one copy of a sequence encoding an H chain of an antibody and at least one copy of a sequence encoding an L chain of the antibody.

The gene of interest may further include a sequence encoding at least one selected from the group consisting of a nucleic acid constituting a viral preparation, a transfer control nucleic acid, and a non-coding RNA. Examples of the non-coding RNA (ncRNA) include microRNA (miRNA), short hairpin RNA (shRNA), small interfering RNA (siRNA), small nuclear RNA (snRNA), ribosomal RNA (rRNA), and transfer RNA (tRNA).

The cell according to the present disclosure may be a prokaryotic cell or a eukaryotic cell. Examples of the prokaryotic cell include a bacterial cell. Examples of the eukaryotic cell include fungi, yeast, insect cells, and mammalian cells. Specific examples of the bacterial cell, the fungi, the yeast, and the insect cells are the same as the specific examples described in the description of the method for producing a cell.

Examples of the mammalian cell include Chinese hamster ovary cells (CHO cells), baby hamster kidney cells (BHK cells), a human embryonic kidney cell line (for example, HEK293 cells), a human retinoblast-derived cell line (for example, PER.C6 cells), a mouse myeloma cell line (for example, NS0 cells and SP2/0 cells), and established cell lines

derived from these cells.

Examples of the CHO cell include a CHO-DG44 cell, a CHO-K1 cell, a CHO-DXB11 cell, a $CHOpro3^{-}$ cell, and an established cell derived from these cells.

Examples of the mammalian cell include cells differentiated from mammalian cells having differentiation potency. For example, the cell is a cell obtained by introducing a gene of interest into a pluripotent stem cell (an ES cell, an iPS cell, or the like) or a pluripotent stem cell (a mesenchymal stem cell, a tissue stem cell, a somatic stem cell, or the like) and then differentiating the cell.

The cell according to the present disclosure has the following forms (A) to (C).

(A) The genome has a region G including one site 1, one site 2, one site 3, and one site 4, which are sites formed by recombination of a recombinase recognition site, in this order.
(B) The site 1 and the site 4 have sequence identity, and the site 2 and the site 3 have sequence identity.
(C) The region G has a gene of interest disposed between the site 1 and the site 2, and a gene of interest disposed between the site 3 and the site 4.

In the present disclosure, the identity of the base sequences of the site 1 to the site 4 means the identity of the base sequences read in the 5' → 3' direction of the DNA strand on the side in the 5' → 3' direction toward the gene of interest adjacent to each site. The reading strand of the site 1 and the reading strand of the site 4 are different DNA strands, and the reading strand of the site 2 and the reading strand of the site 3 are different DNA strands.

The sequence identity of the site 1 and the site 4 is, for example, 80% or more, 90% or more, 95% or more, or 100%.

The sequence identity of the site 2 and the site 3 is, for example, 80% or more, 90% or more, 95% or more, or 100%.

The cell of the present disclosure can be produced by one kind of recombinase and a host genome and a donor vector, which have the forms (1) to (4). The forms (A) to (C) of the cell of the present disclosure are realized by the host genome and the donor vector having the forms (1) to (4).

FIG. 1 shows a form example of the region G produced by the host genome and the donor vector, which have the forms (1) to (4).

In a case where the cell of the present disclosure is a cell produced by one type of recombinase and a host genome and donor vector having forms (1) to (4),

site 1 is a site formed by recombination between RRS1 and RRS5,
site 2 is a site formed by recombination between RRS2 and RRS6,
site 3 is a site formed by recombination between RRS3 and RRS6, and
site 4 is a site formed by recombination between RRS4 and RRS5.

The number of bases in each of sites 1 to 4 may range from 1 bp to 1000 bp, typically from 10 bp to 300 bp, and more typically from 20 bp to 200 bp.

An example of the embodiment of the site 1 to the site 4 is a site formed by recombination of the recognition site of the serine recombinase.

Examples of the serine recombinase include one kind selected from the group consisting of Bxb1, φC31, TP901, A118, SPβc, TG1, φBT1, φRv1, φ370.1, Wβ, Pa01, and Pa03.

An example of the embodiment of the site 1 to the site 4 includes the following forms (a) to (d).

(a) The site 1 and the site 4 have the same two bases in the central portion, and the entire base sequences have identity. The identity of the entire base sequence is, for example, 80% or more, 90% or more, 95% or more, or 100%.
(b) The site 2 and the site 3 have the same two bases in the central portion, and the entire base sequences have identity. The identity of the entire base sequence is, for example, 80% or more, 90% or more, 95% or more, or 100%.
(c) The site 1 (and the site 4) and the site 2 (and the site 3) have one or both of the two bases in the central portion different from each other, and the entire base sequences have identity. The identity of the entire base sequence is, for example, 80% or more, 90% or more, or 95% or more. The site 1 (and the site 4) and the site 2 (and the site 3) may have the same sequence except for one or both of the two bases in the central portion.
(d) The number of bases of each of the site 1 to the site 4 may be in a range of 1 bp to 1,000 bp, generally in a range of 10 bp to 300 bp, and more generally in a range of 20 bp to 200 bp.

An example of the embodiment of the site 1 to the site 4 is that the site 1 and the site 4 have the same sequence, and the site 2 and the site 3 have the same sequence. The cell having the form can be produced by a host genome and a donor vector having the form (1) to the form (4) and the form (5).

An example of the embodiment of the cell according to the present disclosure is further the following form (D).

(D) The transcription direction of the gene of interest disposed between the site 1 and the site 2 is a direction from the site 2 to the site 1, and the transcription direction of the gene of interest disposed between the site 3 and the site 4 is a direction from the site 3 to the site 4.

The form (D) means that the transcription directions of the two gene of interests arranged in the region G are directions (←→) away from each other. In the form in which the transcription directions of the two adjacent gene of interests are in directions (←→) away from each other, the high expression of the gene of interest can be expected as compared with the form in which the transcription directions of the two adjacent gene of interests are in directions (→←) approaching each other.

The cell having the form (D) can be produced by a host genome and a donor vector having the form (1) to the form (4) and the form (6).

The form after recombination shown in FIG. 1 is the form (D). The transcription directions of the two gene of interests arranged in the region G are directions (←→) away from each other.

An example of the embodiment of the cell according to the present disclosure has, in the region G, a selectable marker gene (1) disposed between the site 1 and the site 2, and a selectable marker gene (2) disposed between the site 3 and the site 4. The selectable marker genes (1) and (2) are genes that express a positive selection marker used for the selection and concentration of the cell according to the present disclosure. The cell having the form can be produced by a host genome and a donor vector having the form (1) to the form (4) and the form (8).

Specific examples of the selectable marker genes (1) and (2) are the same as the third selectable marker gene described in the description of the donor vector.

The region G is a continuous region. The cell according to the present disclosure may have one region G or two or more regions G in the entire genome.

In the region G, the number of bases between the outer end of the site 1 and the outer end of the site 4, which is the site farthest from the site 1, is, for example, 100 kbp or less, 70 kbp or less, 50 kbp or less, 30 kbp or less, and 10 kbp or less.

The number of bases between the outer end of the site 1 and the outer end of the site 4 is, for example, 100 bp or more, 1 kbp or more, and 2 kbp or more.

In the region G, the number of bases between the outer end of the site 2 (end close to the site 1) and the outer end of the site 3 (end close to the site 4) is preferably 50 bp or more, more preferably 100 bp or more, and still more preferably 200 bp or more. According to the present form, two gene of interests present in the region G are arranged in close proximity to each other at an appropriate distance, and high expression of the gene of interest can be expected.

<Method for producing protein>

The present disclosure provides a method for producing a protein having excellent productivity. In the method for producing a protein according to the present disclosure, a cell that highly expresses a gene of interest is used, whereby the productivity of a target protein is excellent.

In the method for producing a protein according to the present disclosure, the cell according to the present disclosure is cultured to express a protein encoded by a gene of interest. By culturing the cell, the target protein is produced in the cell, and the target protein is accumulated in the culture solution and/or the cell.

A method for culturing a cell and a culture medium composition may be selected according to the type of the cell. Culture conditions (for example, culture scale, cell density, temperature, and $CO_2$ concentration) may also be selected depending on the type of host cell.

An example of an embodiment of the method for producing a protein according to the present disclosure includes recovering a target protein from a culture solution. Examples of a method for recovering a target substance from a culture solution include centrifugal separation, filtration, diafiltration, ion exchange chromatography, affinity chromatography, hydrophobic interaction chromatography, gel filtration chromatography, and high-performance liquid chromatography (HPLC). The recovered target protein is used, for example, for producing a pharmaceutical composition.

An example of an embodiment of the method for producing a protein according to the present disclosure includes recovering a cell in which a target protein is accumulated from a culture medium. Examples of a method of recovering cells from a culture medium include centrifugation and filtration. The target protein is accumulated inside or on the surface of the cell according to the properties thereof. The recovered cells are, for example, administered, infused, or transplanted into a mammal.

Examples

Hereinafter, the method for producing a cell according to the present disclosure will be described in more detail with reference to specific examples. The materials, the treatment procedures, and the like shown in the following specific examples can be appropriately changed as long as the changes do not depart from the gist of the present disclosure. The

scope of the method for producing a cell according to the present disclosure and the like should not be construed as being limited by the following specific examples.

The base sequences of RRS1 to RRS6 in the following examples are as follows. In each of the following sequences, two bases in the central portion related to whether or not recombination between RRSs is possible are underlined.

RRS1 and RRS4

SEQ ID NO1: 5'-GTCGTGGTTTGTCTGGTCAACCACCGCG GT CTCAGTGGTGTACGGTACAAACCCCGAC-3'

RRS5

SEQ ID NO2: 5'-TCGGCCGGCTTGTCGACGACGGCG GT CTCCGTCGTCAGGATCATCCGGGC-3'

RRS2 and RRS3

SEQ ID NO3: 5'-GTCGTGGTTTGTCTGGTCAACCACCGCG CT CTCAGTGGTGTACGGTACAAACCCCGAC-3'

RRS5

SEQ ID NO4: 5'-TCGGCCGGCTTGTCGACGACGGCG CT CTCCGTCGTCAGGATCATCCGGGC-3'

RRS1 and RRS4 are native attP of Bxb1 recombinase (also known as Bxb1 integrase). RRS5 is native attB of Bxb1 recombinase.

RRS2 and RRS3 are sequences in which two bases "GT" in the central portion of the native attP of the Bxb1 recombinase are modified to "CT". RRS6 is a sequence in which two bases "GT" in the central portion of the native attB of the Bxb1 recombinase are modified to "CT".

<Construction of vector for constructing host genome>

A vector for constructing a host genome was produced by using a custom synthesis service for an artificial gene. Hereinafter, this vector is referred to as "vector A".

The vector A has RRS1 to RRS4, has a first negative selection gene between RRS 1 and RRS2, and has a second negative selection gene between RRS3 and RRS4. The first negative selection gene and the second negative selection gene are a thymidine kinase gene derived from herpes simplex virus.

The vector A has an origin of replication for amplification using Escherichia coli and an ampicillin resistance gene as a selection marker.

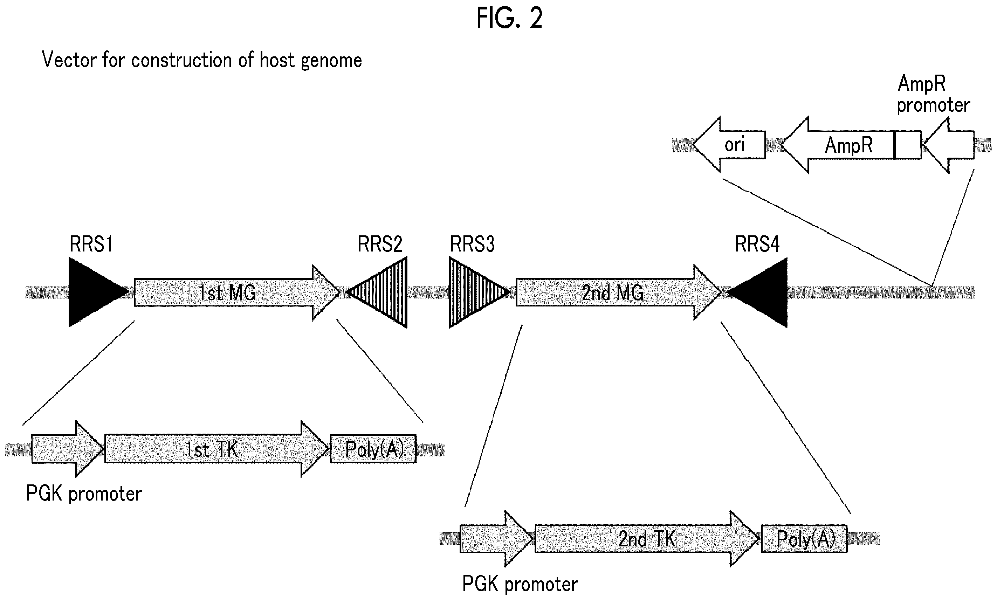

FIG. 2 shows a schematic configuration diagram of the vector A. The order and transcription direction of the genes are as shown in FIG. 2. The orientations of RRS1 to RRS4 are as shown in Table 1. The total length of the vector A is about 8 kbp, the number of bases between the outer end of RRS1 and the outer end of RRS4 is about 4.5 kbp, and the number of bases between the outer end of RRS2 (end close to RRS1) and the outer end of RRS3 (end close to RRS4) is about 300 bp.

<Construction of donor vector>

The following DNA fragment (1) and DNA fragment (2) were synthesized using a custom synthesis service of an artificial gene.

- · DNA fragment (1): red fluorescent protein mCherry gene-puromycin resistance gene All nucleic acids necessary for gene expression are included, and a coding sequence of a 2A self-cleaving peptide is present between the two genes.
- · DNA fragment (2): L chain gene-H chain gene of antibody All nucleic acids necessary for gene expression are

included in each gene of each chain.

A vector in which the DNA fragment (1) and the DNA fragment (2) were linked was produced using an In-Fusion HD Cloning Kit (Takara Bio Inc., product code: 639648). The DNA fragment (2) was further linked to the produced vector to obtain the following DNA fragment (3).

- DNA fragment (3): red fluorescent protein mCherry gene-puromycin resistance gene-L chain gene-H chain gene-L chain gene-H chain gene

A DNA fragment in which RRS5 was added to one end and RRS6 was added to the other end of the DNA fragment (3) was produced by a PCR method, and this DNA fragment was linked to a backbone vector to produce a donor vector. Hereinafter, this vector is referred to as a "donor vector B".

The donor vector B has an antibody gene (L chain gene-H chain gene-L chain gene-H chain gene) as a gene of interest between RRS5 and RRS6.

The donor vector B has a red fluorescent protein mCherry gene-puromycin resistance gene as a selectable marker gene between RRS5 and RRS6.

The donor vector B has an origin of replication for amplification using Escherichia coli and an ampicillin resistance gene as a selectable marker.

Hereinafter, the entire gene group disposed between RRS5 and RRS6 is referred to as "GoI-MG".

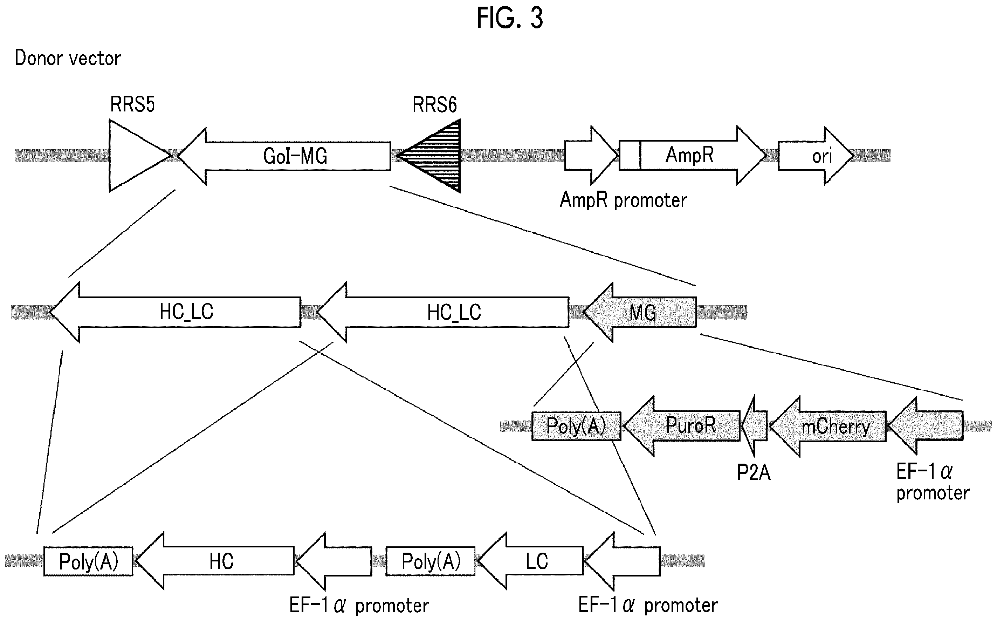

FIG. 3 shows a schematic configuration diagram of the donor vector B. The order and transcription direction of the genes are as shown in FIG. 3. The orientations of RRS5 and RRS6 are as shown in Table 1.

<Construction of Bxb1 expression vector>

An expression vector of Bxb1 recombinase was produced using a custom synthesis service for an artificial gene. Hereinafter, this expression vector is referred to as "vector C".

The vector C has an origin of replication for amplification using Escherichia coli, an ampicillin resistance gene as a selectable marker, and a Bxb1 gene. The Bxb1 gene is a gene in which a codon is optimized for expression in a mammalian cell and a nuclear localization signal sequence derived from SV40 is added to the 5' side.

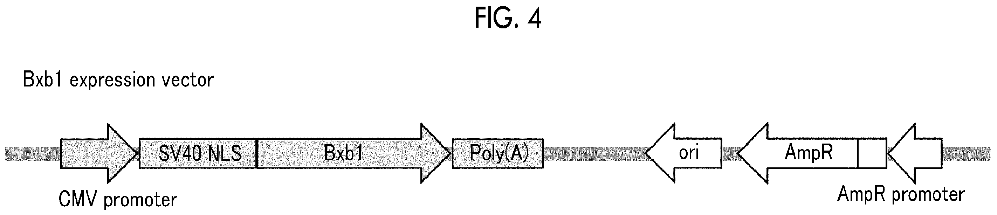

FIG. 4 shows a schematic configuration diagram of the vector C. The order and transcription direction of the genes are as shown in FIG. 4.

<Culture of cells>

As a host cell, CHO-DG44 cells were used.

For maintenance subculture of the CHO-DG44 cells, a liquid culture medium obtained by adding hypoxanthine/thymidine (Thermo Fisher Scientific, HT Supplement (100X)) to a serum-free basal culture medium (Thermo Fisher Scientific, CD OptiCHO Medium) was used. In the cloning experiment of 1 cell, a liquid culture medium obtained by adding 10% (v/v) fetal bovine serum to an IMDM basal culture medium was used.

<Establishment of host cell>

The vector A was introduced into the CHO-DG44 cells by electroporation. This treatment was performed using a 4D-Nucleofector device and an SF Cell Line 4D-Nucleofector X Kit L (Lonza, "Nucleofector" is a registered trademark). The vector A used for the treatment was 11 μg.

After the introduction of the vector A, maintenance subculture of the cells was performed using a subculture medium. On the 6th day of culture, 1 cell was seeded per well in a 96-well plate, and the cells were single-cloned.

Genomes were extracted from the established 24 clones, and using a digital PCR system (Bio-Rad Laboratories, ddPCR Supermix for Probes (No dUTP) #1863024), one clone in which one copy of the vector A was inserted into the genome was acquired. Hereinafter, this clone is referred to as "CHO-159B3 cells".

A region R, which is a region in the genome of the CHO-159B3 cells into which the vector A was inserted, was amplified by PCR, and Sanger sequencing analysis was performed (using the contracted analysis service of Fasmac Co., Ltd.).

From the results of the sequence analysis, it was confirmed that RRS1 to RRS4, the first TK gene, and the second TK gene were present in the region R as designed. That is, the arrangement order of RRS1 to RRS4, the first TK gene, and the second TK gene in the region R was as shown in the schematic view of FIG. 1, and the orientations of RRS1 to RRS4 in the region R were as shown in Table 1. The number of bases between the outer end of RRS1 and the outer end of RRS4 was about 4.5 kbp, and the number of bases between the outer end of RRS2 (end close to RRS1) and the outer end of

RRS3 (end close to RRS4) was about 300 bp.

In a case where the copy numbers of the first TK gene and the second TK gene in the genome of the CHO-159B3 cells (copy numbers of the Txnip gene) were measured, both the first TK gene and the second TK gene were about 1 copy.

<Incorporation of gene of interest into host genome>

The donor vector B and the vector C were introduced into the CHO-159B3 cells by electroporation. This treatment was performed using a 4D-Nucleofector device and an SF Cell Line 4D-Nucleofector X Kit L (Lonza). The donor vector B used in the treatment was 12 μg, and the vector C was 6 μg.

After the introduction of the donor vector B and the vector C, maintenance subculture of the cells was performed using a subculture medium for the purpose of expression and reaction of the Bxb1 recombinase. On the 11th day of culture, 30 cells were seeded per well in a 96-well plate, and selection with ganciclovir and puromycin and visual selection with red fluorescence were performed.

The genome was extracted from the established 21 clones, the region corresponding to the region R was amplified by PCR, and Sanger sequencing analysis was performed. Clones in which a site formed by recombination of the Bxb1 recombinase was present at the positions of the RRS1 and the RRS4 present in the region R were primarily selected.

The clone that was primarily selected was subjected to sequence analysis of the entire length of the region G formed by recombination of the region R and the donor vector B, and one clone in which the sites 1 to 4 and the GoI-MG were present as designed was selected. The sequence analysis was performed using a long-read sequencer MinION Mk1C (Oxford Nanopore Technologies).

The region G of the selected one clone had the sites 1 to 4, the GoI-MG disposed between the site 1 and the site 2, and the GoI-MG disposed between the site 3 and the site 4. The transcription directions of the two GoI-MG's were directions (←→) away from each other. The base sequence of the region G had 99% or more identity with the designed base sequence.

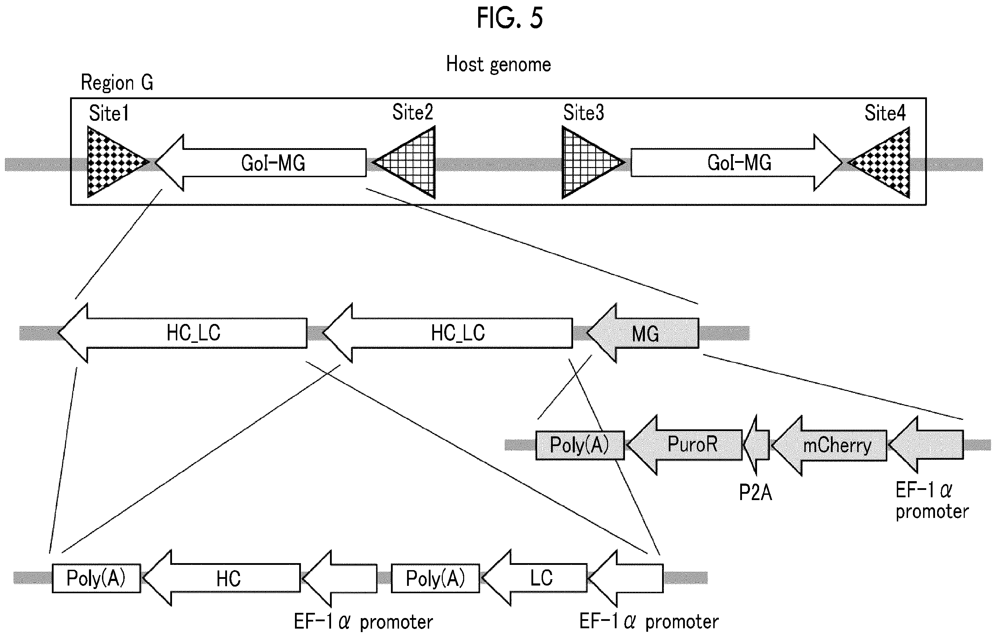

FIG. 5 shows a schematic configuration diagram of the region G of the above-described clone. The order and the transcription direction of the genes are as shown in FIG. 5.

In FIG. 5, the GoI-MG disposed between the site 1 and the site 2 among the two GoI-MG's is described in detail. The GoI-MG disposed between the site 3 and the site 4 includes the same gene group as the GoI-MG disposed between the site 1 and the site 2, but is disposed in the opposite direction.

All documents, patent applications, and technical standards described in the present specification are incorporated herein by reference to the same extent as in a case of being specifically and individually noted that individual documents, patent applications, and technical standards are incorporated herein by reference.

The disclosure of JP2023-202228 filed on November 29, 2023 is incorporated herein by reference in its entirety.

[Sequence Table] International application 23F01164W1JP24041881_1.xml based on International Patent Cooperation Treaty

## Claims

**1.** A method for producing a cell by integrating a gene of interest into a genome of a host cell using one type of recombinase and one type of donor vector, the method comprising:

introducing the donor vector for the gene of interest into the host cell;
causing the recombinase to act in the host cell into which the donor vector has been introduced; and
selecting, from the host cell in which the recombinase has been caused to act, a cell expressing the gene of interest,
wherein the genome of the host cell and the donor vector satisfies the following (1) to (4),

(1) the genome of the host cell has a region R including, in this order, one each of RRS1, RRS2, RRS3, and RRS4, which are recognition sites of the recombinase,
(2) the donor vector has RRS5 and RRS6, which are recognition sites of the recombinase, and the gene of interest disposed between RRS5 and RRS6,
(3) RRS1 and RRS4 are recombinable with RRS5 and not recombinable with RRS6, and
(4) RRS2 and RRS3 are recombinable with RRS6 and not recombinable with RRS5.

**2.** The method for producing a cell according to claim 1,
wherein the genome of the host cell further satisfies the following (5),

(5) RRS1 and RRS4 have an identical sequence, and RRS2 and RRS3 have an identical sequence.

3. The method for producing a cell according to claim 1,
wherein the donor vector further satisfies the following (6),
(6) a transcription direction of the gene of interest disposed between RRS5 and RRS6 is a direction from RRS6 toward RRS5.

4. The method for producing a cell according to claim 1,
wherein the genome of the host cell further satisfies the following (7),
(7) the region R includes a first selectable marker gene disposed between RRS1 and RRS2 and a second selectable marker gene disposed between RRS3 and RRS4.

5. The method for producing a cell according to claim 4,
wherein the donor vector further satisfies the following (8),
(8) the donor vector includes a third selectable marker gene disposed between RRS5 and RRS6.

6. The method for producing a cell according to claim 1, further comprising:
introducing an expression vector of the recombinase into the host cell.

7. The method for producing a cell according to claim 1,
wherein the recombinase is a serine recombinase.

8. The method for producing a cell according to claim 1,
wherein the host cell is a mammalian cell.

9. The method for producing a cell according to claim 1,
wherein the host cell is a CHO cell.

10. The method for producing a cell according to any one of claims 1 to 9,
wherein the gene of interest is a gene encoding at least one selected from the group consisting of an enzyme, an antibody, an interleukin, a cytokine, a chemokine, a hormone, a growth factor, a transcription factor, a receptor, a viral preparation, a vaccine, a therapeutic protein, subunits thereof, and fragments thereof.

11. A cell in which a gene of interest is integrated into a genome,
wherein the cell satisfies the following (A) to (C),

(A) the genome has a region G including, in this order, one each of site 1, site 2, site 3, and site 4, the sites being sites formed by recombination of recognition sites of a recombinase,
(B) site 1 and site 4 have sequence identity, and site 2 and site 3 have sequence identity, and
(C) the region G includes the gene of interest disposed between site 1 and site 2 and the gene of interest disposed between site 3 and site 4.

12. The cell according to claim 11,
wherein the cell satisfies the following (D),
(D) a transcription direction of the gene of interest disposed between site 1 and site 2 is a direction from site 2 toward site 1, and a transcription direction of the gene of interest disposed between site 3 and site 4 is a direction from site 3 toward site 4.

13. The cell according to claim 11,
wherein the recombinase is a serine recombinase.

14. The cell according to claim 11,
wherein the cell is a mammalian cell.

15. The cell according to claim 11,
wherein the cell is a CHO cell.

16. The cell according to claim 11,

wherein the gene of interest is a gene encoding at least one selected from the group consisting of an enzyme, an antibody, an interleukin, a cytokine, a chemokine, a hormone, a growth factor, a transcription factor, a receptor, a viral preparation, a vaccine, a therapeutic protein, subunits thereof, and fragments thereof.

**17.** A method for producing a protein, the method comprising:
culturing the cell according to any one of claims 11 to 16 to express a protein encoded by the gene of interest.

Host genome
Region R
RRS1
1st MG
RRS2
RRS3
2nd MG
RRS4
RRS5
GoI
RRS6
Donor vector
RRS5
GoI
RRS6
Donor vector
Host genome
Region G
Site1
GoI
Site2
Site3
GoI
Site4

FIG. 1

FIG. 2

Vector for construction of host genome

AmpR promoter
ori
AmpR
RRS1
RRS2
RRS3
RRS4
1st MG
2nd MG
1st TK
Poly(A)
PGK promoter
2nd TK
Poly(A)
PGK promoter

FIG. 3

Donor vector
RRS5
GoI-MG
RRS6
AmpR
ori
AmpR promoter
HC_LC
HC_LC
MG
Poly(A)
PuroR
mCherry
P2A
EF-1α promoter
Poly(A)
HC
Poly(A)
LC
EF-1α promoter
EF-1α promoter

Bxb1 expression vector
CMV promoter
SV40 NLS
Bxb1
Poly(A)
ori
AmpR
AmpR promoter

FIG. 4

FIG. 5
Host genome
Region G
Site1
Site2
Site3
Site4
GoI-MG
GoI-MG
HC_LC
HC_LC
MG
Poly(A)
PuroR
P2A
mCherry
EF-1α promoter
Poly(A)
HC
EF-1α promoter
Poly(A)
LC
EF-1α promoter

## INTERNATIONAL SEARCH REPORT

International application No. **PCT/JP2024/041881**

**A. CLASSIFICATION OF SUBJECT MATTER**

***C12N 15/90***(2006.01)i; ***C12N 5/10***(2006.01)i; ***C12P 21/00***(2006.01)i; *C12N 15/12*(2006.01)n; *C12N 15/85*(2006.01)n
FI: C12N15/90 100Z; C12N5/10 ZNA; C12P21/00 C; C12N15/85 Z; C12N15/12

According to International Patent Classification (IPC) or to both national classification and IPC

**B. FIELDS SEARCHED**

Minimum documentation searched (classification system followed by classification symbols)

C12N15/90; C12N5/10; C12P21/00; C12N15/12; C12N15/85

Documentation searched other than minimum documentation to the extent that such documents are included in the fields searched

Published examined utility model applications of Japan 1922-1996
Published unexamined utility model applications of Japan 1971-2025
Registered utility model specifications of Japan 1996-2025
Published registered utility model applications of Japan 1994-2025

Electronic data base consulted during the international search (name of data base and, where practicable, search terms used)

JSTPlus/JMEDPlus/JST7580 (JDreamIII); CAplus/MEDLINE/EMBASE/BIOSIS (STN)

**C. DOCUMENTS CONSIDERED TO BE RELEVANT**

| Category* | Citation of document, with indication, where appropriate, of the relevant passages | Relevant to claim No. |
|---|---|---|
| X<br>Y | WO 2023/129974 A1 (BRISTOL-MYERS SQUIBB COMPANY) 06 July 2023 (2023-07-06)<br>claims, paragraph [0350], example 4, fig. 12A | 1-17<br>1-17 |
| X | CHENG, Shu-Yun et al., Integration of Multiple Phage Attachment Sites System to Create the Chromosomal T7 System for Protein Production in Escherichia coli Nissle 1917, Journal of Agricultural and Food Chemistry, 2022, vol. 70, pp. 10239-10247<br>p. 10242, right column, last paragraph - p. 10245, left column, first paragraph, fig. 2, 5 | 11, 13-17 |
| Y | GHOSH, Pallavi et al., The Orientation of Mycobacteriophage Bxb1 Integration Is Solely Dependent on the Central Dinucleotide of attP and attB, Molecular Cell, 2003, vol. 12, pp. 1101-1111<br>p. 1105, right column, last paragraph - p. 1106, right column, first paragraph | 1-17 |
| A | JP 2004-516852 A (COMMONWEALTH SCIENTIFIC AND INDUSTRIAL RESEARCH ORGANISATION) 10 June 2004 (2004-06-10)<br>fig. 1 | 1-17 |

☑ Further documents are listed in the continuation of Box C. ☑ See patent family annex.

* Special categories of cited documents:
"A" document defining the general state of the art which is not considered to be of particular relevance
"D" document cited by the applicant in the international application
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **03 February 2025** | **10 February 2025** |

| Name and mailing address of the ISA/JP | Authorized officer |
|---|---|
| **Japan Patent Office (ISA/JP)**<br>**3-4-3 Kasumigaseki, Chiyoda-ku, Tokyo 100-8915**<br>**Japan** | Telephone No. |

Form PCT/ISA/210 (second sheet) (July 2022)

INTERNATIONAL SEARCH REPORT

International application No.
**PCT/JP2024/041881**

**C. DOCUMENTS CONSIDERED TO BE RELEVANT**

| Category* | Citation of document, with indication, where appropriate, of the relevant passages | Relevant to claim No. |
|---|---|---|
| A | US 2004/0115814 A1 (PROTEIN DESIGN LABS, INC.) 17 June 2004 (2004-06-17) fig. 5 | 1-17 |

Form PCT/ISA/210 (second sheet) (July 2022)

**INTERNATIONAL SEARCH REPORT**

International application No.
**PCT/JP2024/041881**

**Box No. I Nucleotide and/or amino acid sequence(s) (Continuation of item 1.c of the first sheet)**

1. With regard to any nucleotide and/or amino acid sequence disclosed in the international application, the international search was carried out on the basis of a sequence listing:
   a. ☑ forming part of the international application as filed.
   b. ☐ furnished subsequent to the international filing date for the purposes of international search (Rule 13*ter*.1(a)),
      ☐ accompanied by a statement to the effect that the sequence listing does not go beyond the disclosure in the international application as filed.

2. ☐ With regard to any nucleotide and/or amino acid sequence disclosed in the international application, this report has been established to the extent that a meaningful search could be carried out without a WIPO Standard ST.26 compliant sequence listing.

3. Additional comments:

Form PCT/ISA/210 (continuation of first sheet) (July 2022)

**INTERNATIONAL SEARCH REPORT**
**Information on patent family members**

International application No.
**PCT/JP2024/041881**

| Patent document cited in search report | Publication date (day/month/year) | Patent family member(s) | Publication date (day/month/year) |
|---|---|---|---|
| WO 2023/129974 A1 | 06 July 2023 | EP 4457342 A1 | |
| | | KR 10-2024-0128067 A | |
| | | JP 2025-501221 A | |
| JP 2004-516852 A | 10 June 2004 | US 2003/0049835 A1<br>fig. 1 | |
| | | WO 2002/059294 A1 | |
| | | EP 1354035 A1 | |
| US 2004/0115814 A1 | 17 June 2004 | WO 2004/029284 A2<br>fig. 5 | |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- EP 2711428 A
- WO 2017184831 A
- WO 2017184832 A
- WO 2020072480 A
- JP 2023202228 A

### Non-patent literature cited in the description

- *Molecular Cell*, 2003, vol. 12, 1101-1111
- *BMC Biotechnology*, 2013, vol. 13, 87
- *Acta Biochim Biophys Sin*, 2017, vol. 49 (1), 44-50
- *J. Mol. Biol.*, 1986 (1), 113-30
- *J. Mol. Biol.*, 1986, vol. 189 (1), 113-30